# EUROPEAN PATENT APPLICATION

(11) **EP 4 712 091 A1**
(43) Date of publication of application: **18.03.2026**
(21) Application number: 24802878.9
(22) Date of filing: 30.04.2024
(51) Int. Cl.: G16H 10/60

(54) **MEDICAL DATA DISPLAY METHOD, APPARATUS, DEVICE, AND STORAGE MEDIUM**

(30) Priority: 09.05.2023 CN 202310520863
(71) Applicant: Shining 3D Tech Co., Ltd., Hangzhou, Zhejiang 311258 (CN)
(72) Inventor: JIANG, Tengfei, Hangzhou, Zhejiang 311258 (CN); ZHAO, Xiaobo, Hangzhou, Zhejiang 311258 (CN); ZHANG, Jian, Hangzhou, Zhejiang 311258 (CN); WANG, Jialei, Hangzhou, Zhejiang 311258 (CN); LIN, Zhongwei, Hangzhou, Zhejiang 311258 (CN)
(74) Representative: Dai, Simin
(86) International application number: PCT/CN2024/090864
(87) International publication number: WO 2024/230596

(57) **Abstract**

A medical data display method, an apparatus, a device, and a storage medium. The method comprises: after acquiring multiple paths of medical data collected by multiple device sources in respect of the same target subject at different time nodes, aligning in space multiple paths of medical data collected by different device sources at the same time node, so as to unify same into one coordinate system; and aligning in space multiple paths of medical data collected by the same device source at different time nodes, so as to unify same into one coordinate system. Thus, the present invention can display on an interaction interface multiple paths of medical data collected by different device sources at a certain time node, and the change conditions of medical data collected by a certain device source at different time nodes, thereby improving data presentation efficiency and facilitating users in checking data.

## Description

### CROSS-REFERENCE TO RELATED APPLICATIONS

The present application claims priority to Chinese patent application No. 202310520863.0, filed with the China National Intellectual Property Administration on May 9, 2023, entitled "Medical data display method and apparatus, device, and storage medium", which is hereby incorporated by reference in its entirety.

### TECHNICAL FIELD

The present disclosure relates to the field of data processing technologies, and in particular, to a medical data display method and apparatus, a device, and a storage medium.

### BACKGROUND

During a process of diagnosing and treating a patient, it is usually necessary to collect data from a same part of the patient using multiple medical devices, in order to diagnose the patient based on multiple streams of medical data collected from the multiple medical devices. Meanwhile, for a same type of medical data from the same patient, doctors also need to view the changes in this medical data over time.

Currently, medical data collected by different medical devices need to be viewed through different software platforms. When doctors view medical data collected by multiple medical devices, they need to constantly switch between different software platforms, which is very cumbersome and greatly affects work efficiency. Additionally, current medical data viewing software cannot display the changes in medical data collected by the same medical device, which is not conducive to doctors' analysis of changes in medical data.

### SUMMARY

The present disclosure provides a medical data display method and apparatus, a device and a storage medium.

According to a first aspect of the embodiments of the present disclosure, a medical data display method is provided, including: acquiring multiple streams of medical data collected by multiple device sources for a same target object at different time points respectively; spatially aligning multiple streams of medical data collected by different device sources at a same time point, and spatially aligning multiple streams of medical data collected by a same device source at different time points; in response to acquiring an instruction triggered by a user for displaying medical data at a target time point, displaying, on an interactive interface, aligned multiple streams of medical data collected by different device sources at the target time point; and in response to acquiring an instruction triggered by the user for displaying medical data collected by a target device source, displaying, on the interactive interface, changes in medical data collected by the target device source at different time points.

According to a second aspect of the embodiments of the present disclosure, a medical data display apparatus is provided, including: an acquiring module, configured to acquire multiple streams of medical data collected by multiple device sources for a same target object at different time points respectively; an aligning module, configured to spatially align multiple streams of medical data collected by different device sources at a same time point, and spatially align multiple streams of medical data collected by a same device source at different time points; a display module, configured to, in response to acquiring an instruction triggered by a user for displaying medical data at a target time point, display, on an interactive interface, aligned multiple streams of medical data collected by different device sources at the target time point; and in response to acquiring an instruction triggered by the user for displaying medical data collected by a target device source, display, on the interactive interface, changes in medical data collected by the target device source at different time points.

According to a third aspect of the embodiments of the present disclosure, an electronic device is provided, including one or more processors, a memory, and a computer instruction stored in the memory and executable by the one or more processors, where when executing the computer instruction, the one or more processors are caused to perform the method according to the above first aspect.

According to a fourth aspect of the embodiments of the present disclosure, a computer-readable storage medium is provided, storing a computer instruction, and when the computer instruction is executed, the method according to the above first aspect is implemented.

In the embodiments of the present disclosure, after the multiple streams of medical data collected by the multiple device sources for the same target object at different time points are acquired, the multiple streams of medical data collected by different device sources at the same time point may be spatially aligned and unified into a same coordinate system, and the multiple streams of medical data collected by the same device source at different time points may be spatially aligned and unified into a same coordinate system, so that the multiple streams of medical data collected by different device sources at a certain time point may be displayed on the interactive interface, and changes in medical data collected by a certain device source at different time points may be displayed.

It is to be understood that both the foregoing general description and the following detailed description are examples and explanatory only and do not intend to limit the present disclosure.

### BRIEF DESCRIPTION OF DRAWINGS

The accompanying drawings, which are incorporated in and constitute a part of this specification, illustrate embodiments consistent with the present disclosure and, together with the description, serve to explain the technical solutions of the present disclosure.
FIG. 1 is a schematic diagram showing collecting of medical data using multiple device sources in an oral diagnosis and treatment scenario according to an embodiment of the present disclosure.
FIG. 2 is a flowchart of a medical data display method according to an embodiment of the present disclosure.
FIG. 3 is a schematic diagram of three-dimensional data alignment according to an embodiment of the present disclosure.
FIG. 4 is a schematic diagram of a user interaction interface according to an embodiment of the present disclosure.
FIG. 5 is a flowchart of a medical data display apparatus according to an embodiment of the present disclosure.
FIG. 6 is a schematic diagram of a logical structure of a device according to an embodiment of the present disclosure.

### DETAILED DESCRIPTION

Reference will now be made in detail to example embodiments, examples of which are illustrated in the accompanying drawings. When the following description refers to the accompanying drawings, unless otherwise indicated, same numbers in different drawings indicate same or similar elements. The implementations described in the following example embodiments do not represent all implementations consistent with the present disclosure. Rather, they are merely examples of apparatuses and methods consistent with some aspects of the present disclosure as detailed in the appended claims.

The terms used in the present disclosure are only for the purpose of describing specific embodiments, and are not intended to limit the present disclosure. As used in the present disclosure and the appended claims, the singular forms "a", "an" and "the" are intended to include the plural forms as well, unless the context clearly indicates otherwise. It should also be understood that the term "and/or" used herein refers to and includes any or all possible combinations of one or more associated listed items. In addition, the term "at least one" herein means any one of a plurality or any combination of at least two of a plurality.

It should be understood that although the terms "first", "second", "third", etc. may be used in the present disclosure to describe various information, these information should not be limited to these terms. These terms are only used to distinguish the same type of information from each other. For example, without departing from the scope of the present disclosure, "first information" may also be referred to as "second information", and similarly, "second information" may also be referred to as "first information". Depending on the context, the word "if" as used herein may be interpreted as "when" or "upon" or "in response to determining".

In order to enable those skilled in the art to better understand the technical solutions in the embodiments of the present disclosure and make the above objects, features and advantages of the embodiments of the present disclosure more obvious and understandable, the technical solutions in the embodiments of the present disclosure will be further described in detail below with reference to the accompanying drawings.

During a process of diagnosing and treating a patient, it is usually necessary to collect data from a same part of the patient using multiple medical devices, in order to diagnose the patient based on multiple streams of medical data collected from the multiple medical devices. Meanwhile, for a same type of medical data from the same patient, doctors also need to view the changes in this medical data over time.

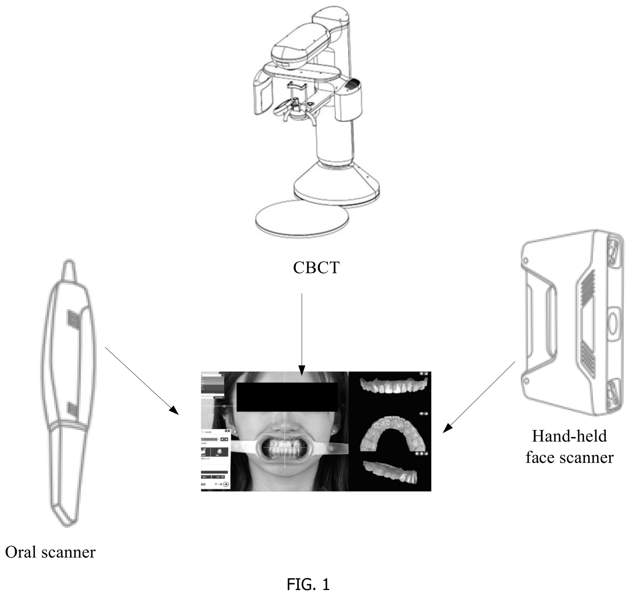

Taking the diagnosis and treatment of oral diseases as an example, the diagnosis and treatment of oral diseases usually involves the collection and analysis of medical data from multiple sources across time, as shown in FIG. 1, these medical data come from different device sources (such as oral scanner, facial scanner, Cone-Beam Computed Tomography (CBCT), Computed Tomography (CT), etc.), and data types are highly diverse, including two-dimensional data, three-dimensional data, pathological data, etc. For example, the data involved in the oral disease diagnosis and treatment process includes different types of medical data: two-dimensional image data such as cephalometric lateral films, panoramic films, periapical films, facial high-definition photographs, tooth high-definition photographs, etc.; three-dimensional data such as tooth three-dimensional scanning data, facial three-dimensional scanning data, skull CBCT data, tooth CBCT data, etc.; pathological data such as cell slice data, blood test reports; and mandibular bone movement data.

Currently, when viewing medical data collected by different devices, doctors need to switch to different software platforms for viewing. For example, the oral three-dimensional scanning data collected by an oral scanner needs to be viewed by software matched with the oral scanner, the facial three-dimensional scanning data collected by the facial scanner needs to be viewed by software matched with the facial scanner, the skull CBCT data and the tooth CBCT data need to be viewed by software matched with the CBCT device, and the pathological data needs to be viewed by other software. However, in most scenarios, these medical data have a certain association, and the doctor needs to compare and analyze these medical data at the same time, which will cause the doctor to constantly switch between multiple software platforms to view different medical data.

In addition, for oral disease diagnosis and treatment scenarios, doctors usually need to view changes in some medical data over time, for example, changes in a tooth morphology over age, and such function cannot be achieved by current data viewing software, which makes it very inconvenient for doctors to perform data analysis and greatly affects work efficiency.

It can be seen that for scenarios such as oral disease diagnosis and treatment involving cross-time and multi-device source data analysis, there is an urgent need for a solution that can facilitate users to perform multi-source data analysis and comparison.

In view of this, embodiments of the present disclosure provide a medical data display method, in which after the multiple streams of medical data collected by the multiple device sources for the same target object at different time points are acquired, the multiple streams of medical data collected by different device sources at the same time point may be spatially aligned and unified into a same coordinate system, and the multiple streams of medical data collected by the same device source at different time points may be spatially aligned and unified into a same coordinate system, so that the multiple streams of medical data collected by different device sources at a certain time point may be displayed on the interactive interface, and changes in medical data collected by a certain device source at different time points may be displayed, which improves data display efficiency, and can be easily viewed by the user.

The medical data display method provided by the embodiments of the present disclosure may be implemented by self-developed software codes with a data viewing function. For example, in some scenarios, the software code may be presented in a form of data viewing software, or may be presented in a form of a web client having a data viewing function, a user may import multiple streams of medical data into the data viewing software or the web client, and the data viewing software or the web client may process and display the imported medical data.

Certainly, in some scenarios, the software code may also be presented in a form of a medical data management system, the medical data management system may be communicatively connected to multiple medical device sources, medical data is obtained from the multiple medical device sources, and the user may manage and view the medical data by using the medical data management system.

The medical data mentioned in the embodiments of the present disclosure may be various types of data required in the diagnosis and treatment process. Taking the oral diagnosis and treatment scenario as an example, the medical data may include: cephalometric lateral films, panoramic films, periapical films, facial high-definition photographs, tooth high-definition photographs; tooth three-dimensional scanning data, facial three-dimensional scanning data, skull CBCT data, tooth CBCT data; cell slice data, blood test reports; and mandibular bone movement data.

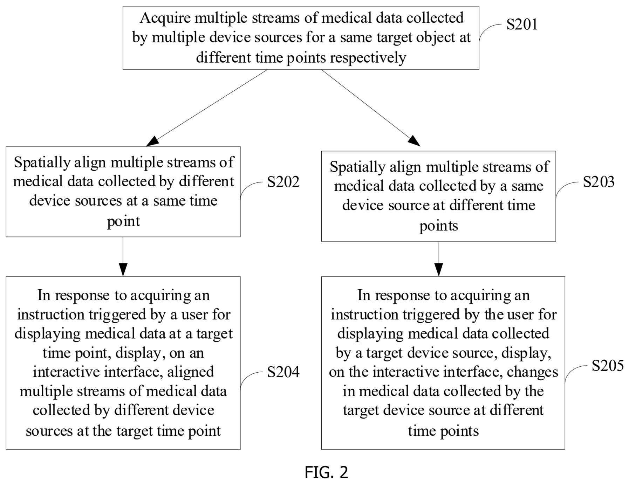

As shown in FIG. 2, FIG. 2 is a schematic diagram of a medical data display method according to an embodiment of the present disclosure.

In step S201, multiple streams of medical data collected by multiple device sources for a same target object at different time points respectively are acquired. For example, the method is performed by a data viewing software, the user may import, into the data viewing software, multiple streams of medical data collected by multiple device sources for a same patient at different time points. Alternatively, for example, the method is performed by a medical data management system, the medical data management system may be communicatively connected to multiple device sources, the multiple device sources may automatically transmit medical data of a patient to the medical data management system after collecting the medical data, and the medical data management system may classify the obtained data based on a user dimension. When data needs to be displayed, the user may select medical data to be displayed in the data management system, and then the medical data selected by the user is processed and displayed.

In step S202, after the multiple streams of medical data are acquired, the multiple streams of medical data collected by different device sources at a same time point may be spatially aligned. Considering that multiple streams of medical data collected by different device sources usually need to be viewed and analyzed together, and medical data collected by different device sources usually have different coordinate systems, after the multiple streams of medical data are acquired, the multiple streams of medical data may be first divided based on a time dimension, for example, medical data collected on a same day is divided into one group, so that multiple streams of data collected by different device sources at each time point can be obtained. Then, the multiple streams of medical data collected by different device sources at the same time point may be spatially aligned and unified to a same coordinate system.

Considering that there are usually overlapping regions in medical data collected by different device sources for a same target object, two streams of data may be matched and aligned by using these overlapping regions. For example, taking an oral diagnosis and treatment scenario as an example, an oral cavity scanner is usually used to scan an oral cavity of the user to obtain three-dimensional scanning data of a user's oral cavity, and at the same time, the CBCT data of the user's oral cavity is also collected by using the CBCT. As the two groups of three-dimensional data both include user's teeth, the two groups of three-dimensional data may be matched based on the teeth, and the two groups of three-dimensional data are unified into a same coordinate system.

In step S203, considering that the user usually also needs to view changes in data of a same dimension at different time points, after the multiple streams of medical data are acquired, the multiple streams of medical data may be first divided according to a device dimension, medical data collected by a same device source is divided into one group, and then multiple streams of medical data collected by the same device source at different time points may be spatially aligned.

After the multiple streams of medical data are aligned, in response to detecting a data display instruction triggered by the user through the interactive interface, the aligned medical data may be displayed based on the data display instruction. For example, the user may select, on the interaction interface, data of which device source is to be displayed at present, or data at which time point is to be displayed. In step 204, in response to acquiring an instruction triggered by the user for displaying medical data at a target time point, the aligned multiple streams of medical data collected by different device sources at the target time point are displayed on an interactive interface.

In step 205, in response to acquiring an instruction triggered by the user for displaying medical data collected by a target device source, changes in medical data collected by the target device source at different time points are displayed on the interactive interface.

For example, it is assumed that the multiple streams of medical data include data X1, X2, and X3 collected by a device source A at time point 1, time point 2, and time point 3 respectively; data Y1, Y2, and Y3 collected by a device source B at time point 1, time point 2, and time point 3 respectively; and data Z1, Z2, and Z3 collected by a device source C at time point 1, time point 2, and time point 3 respectively. The data X1, Y1, and Z1 collected by different device sources at the time point 1 may be aligned, and the user may select to display the aligned X1, Y1, and Z1 at the time point 1, or may select to display any two of the aligned X1, Y1, and Z1. Certainly, X1, X2, and X3 collected by the device source A at three time points, i.e., the time point 1, the time point 2, and the time point 3, may also be aligned, to show changes in data collected by the device source at different time points.

In some embodiments, considering that the medical data acquired for the same target object at the same time point by different device sources usually have overlapping portions, when displaying the aligned medical data acquired by different device sources at the same time point, the overlapping portions in the aligned multiple streams of medical data may be performed with fusion processing first, and then the fused multiple streams of medical data are displayed on the interactive interface. By fusing the overlapping regions and then displaying them, it is possible to avoid that the overlapping portions of the data collected by different device sources are messy.

For example, taking oral disease diagnosis and treatment as an example, assuming that the data collected at the same time point includes oral three-dimensional scanning data, facial three-dimensional scanning data, and oral CBCT data, as there is an overlapping portion in the three groups of data, for example, there are several overlapping teeth in the three-dimensional scanning data of the oral cavity and the three-dimensional scanning data of the face, and there are also overlapping teeth in the three-dimensional scanning data of the oral cavity and the CBCT data of the oral cavity, thus the data can be aligned first to be unified into the same coordinate system, and then the overlapping portions are fused as one stream of data for displaying. In this way, it can be avoided that the finally-presented data is messy due to certain errors in the three streams of data, which would affect the display effect.

In some embodiments, in order to better display changes in the medical data collected by the same device source at different time points, the changes in the medical data collected by the same device source at different time points may be displayed by using animation. Because the collection process is discrete, an animation frame corresponding to a time point between two adjacent time points may be generated in a manner of interpolation animation. For example, continuous animation frames may be generated by using a Morphing technology, to continuously present data changes between adjacent time points.

In some embodiments, considering that the user needs to view the change in the medical data over time from a finer granularity, semantic segmentation processing may also be performed on the medical data collected by the same device source at each time point to obtain multiple semantic entities at each time point. Then, the semantic entities are spatially aligned, that is, a correspondence between the semantic entities is established. When the instruction triggered by the user for displaying the target semantic entity is acquired, the change in the target semantic entity at different time points may be displayed on the interactive interface.

For example, taking an oral disease diagnosis and treatment scenario as an example, three-dimensional data of a user's oral cavity is usually collected, and as a doctor usually needs to view a change in a single tooth over time, the three-dimensional data of the user's oral cavity may be semantically segmented to obtain three-dimensional data of the single tooth. Then, the tooth at different time points is aligned, that is, the tooth with a same number have a corresponding relationship. When the user wishes to view a change in a tooth over time, a corresponding display instruction may be triggered, and after the instruction is detected, the change in the tooth over time may be displayed on the interactive interface. For example, the change in the tooth may be displayed by using animation.

Certainly, in some scenarios, before semantic segmentation is performed on the three-dimensional data, texture mapping processing may be first performed, by using at least one two-dimensional image, on the three-dimensional model represented by the three-dimensional data, to obtain a three-dimensional model carrying texture information, and then semantic segmentation processing is performed, so that an obtained single semantic entity includes the texture information. When a change in a single semantic entity over time is displayed, a change in a semantic entity carrying texture information may also be displayed.

In some embodiments, considering scenarios similar to oral disease diagnosis and treatment, there are many types of data to be analyzed, such as multiple streams of three-dimensional data, multiple streams of two-dimensional image data, pathological data, etc. Therefore, when multiple streams of medical data collected by different device sources at a same time point are spatially aligned, there may be multiple cases, for example, two streams of three-dimensional data are spatially aligned, two two-dimensional images are spatially aligned, at least one two-dimensional image is aligned with three-dimensional data, and pathological data is aligned with at least one two-dimensional image or three-dimensional data, etc. Aligning the two-dimensional image with the three-dimensional data refers to performing texture mapping processing on the three-dimensional model represented by the three-dimensional data by using the two-dimensional image, that is, mapping texture of the two-dimensional image onto the three-dimensional model represented by the three-dimensional data. Aligning the pathological data with the two-dimensional image means determining an image region of the two-dimensional image corresponding to the pathological data, and aligning the pathological data with the three-dimensional data means determining a region of the three-dimensional model represented by the three-dimensional data corresponding to the pathological data.

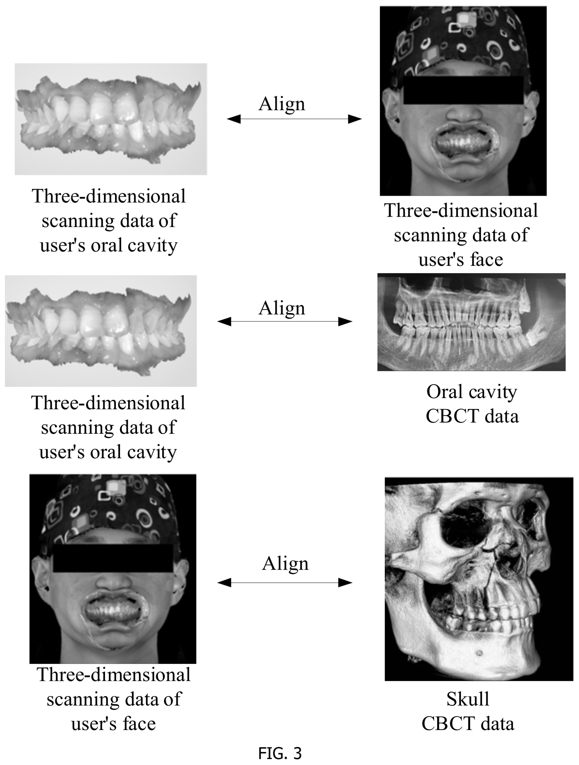

In some embodiments, the multiple streams of medical data are multiple streams of medical data in an oral diagnosis and treatment scenario, as shown in FIG. 3, aligning the three-dimensional data with the three-dimensional data includes at least one of: spatially aligning the three-dimensional scanning data of the user's oral cavity and the three-dimensional scanning data of a user's face, spatially aligning the three-dimensional scanning data of the user's oral cavity and the CBCT data of the user's oral cavity, and spatially aligning the three-dimensional scanning data of the user's face and the CBCT data of a user's skull. In an oral diagnosis and treatment scenario, three-dimensional scanning data of the user's oral cavity, three-dimensional scanning data of the user's face, CBCT data of the user's oral cavity, and CBCT data of the user's skull are usually collected from multiple streams of three-dimensional data, and by synthesizing the multiple streams of data, an internal structure of the user's oral cavity can be learned, so that a treatment plan can be conveniently formulated for a patient, thus the multiple steams of data need to be aligned and displayed.

In some embodiments, the multiple streams of medical data are multiple streams of medical data in the oral diagnosis and treatment scene, and aligning the two-dimensional image with the three-dimensional data (that is, mapping texture of the two-dimensional image data onto the three-dimensional model represented by the three-dimensional data) includes at least one of: mapping texture of the two-dimensional image of the user's face to the three-dimensional model represented by the three-dimensional scanning data of the user's face, and mapping texture of the two-dimensional image of the user's oral cavity to the three-dimensional model represented by the three-dimensional scanning data of the user's oral cavity. In an oral diagnosis and treatment scenario, three-dimensional scanning is usually performed on the user's oral cavity to obtain a three-dimensional model of the oral cavity, and three-dimensional scanning is also performed on the user's face to obtain a three-dimensional model of the user's face. As the three-dimensional model only includes spatial information and does not include texture information, it is also necessary to collect two-dimensional images of the oral cavity and face, and texture of the two-dimensional images needs to be mapped onto the corresponding three-dimensional model to obtain a three-dimensional model of the oral cavity or face with texture information.

In some embodiments, considering that the two-dimensional images in the multiple streams of medical data may include at least two two-dimensional images acquired in different illumination environments, as acquiring environments of the at least two two-dimensional images are inconsistent, the overall brightness of the at least two two-dimensional images is inconsistent, and if the texture of the two two-dimensional images are directly mapped to the corresponding three-dimensional model, the brightness of different parts of the overall three-dimensional model finally presented may be uneven, which affects the final presentation effect. Therefore, in this case, the at least two two-dimensional images may be pre-processed first to unify brightness of the at least two two-dimensional images, and then texture of the at least two pre-processed two-dimensional images are mapped to the corresponding three-dimensional model.

In some embodiments, the at least two two-dimensional images include a two-dimensional image of the user's oral cavity and a two-dimensional image of the user's face. For example, in an oral medical diagnosis scenario, three-dimensional data is usually collected for the user's oral cavity and the user's face to obtain respective three-dimensional models. At the same time, two-dimensional images may also be collected for the user's oral cavity and the user's face respectively, and texture of the two-dimensional images are mapped onto the corresponding three-dimensional models to obtain three-dimensional models with texture information. As the internal of the oral cavity is usually darker than the face, the overall brightness of the two-dimensional image of the user's oral cavity is usually darker than the two-dimensional image of the user's face. If the texture of the two two-dimensional images is directly mapped onto the corresponding three-dimensional models, and then the oral three-dimensional model and the facial three-dimensional model are aligned and then fused for display, the brightness of different parts of the finally fused three-dimensional model is uneven, which affects the overall effect. Therefore, the two two-dimensional images may be pre-processed before texture mapping, to unify brightness of the two two-dimensional images. For example, for an image with relatively dark brightness, the overall brightness of the image can be improved.

In some embodiments, considering that the multiple streams of medical data may include at least two two-dimensional images collected by photographing devices with different resolutions, because the at least two two-dimensional images have different resolutions, after texture of the at least two two-dimensional images are mapped onto the corresponding three-dimensional model, texture information resolutions of different parts in a finally generated overall model are inconsistent, for example, some parts are clear, and some parts are blurred, which affects an overall visual effect. To avoid the foregoing problem, before texture of the at least two two-dimensional images are mapped onto the corresponding three-dimensional models, the at least two two-dimensional images may be pre-processed to unify resolutions of the at least two two-dimensional images.

In some embodiments, the at least two two-dimensional images include a two-dimensional image of a specified part collected by a single-lens reflex camera and a two-dimensional image of the specified part collected by a three-dimensional scanning device, where the specified part includes the user's face and/or the user's oral cavity.

For example, in an oral diagnosis and treatment process, a three-dimensional scanning device is usually used to collect three-dimensional data of the user's oral cavity and/or the user's face, where the three-dimensional scanning device may use two cameras to collect two-dimensional images of the user's oral cavity and/or user's face from different viewing angles, and then use these two-dimensional images to perform three-dimensional reconstruction to obtain a three-dimensional model. Meanwhile, these two-dimensional images may also be used to perform texture mapping processing on the three-dimensional model to obtain a three-dimensional model with texture information. In addition, for the user's oral cavity and/or the user's face, a single-lens reflex camera may also be used to capture a high-definition image, for example, a high-definition image is usually captured for the user's face. As the resolution of the high-definition facial image captured by the single-lens reflex camera is usually higher than the resolution of the oral cavity image captured by the three-dimensional scanning device, if texture of the two two-dimensional images is mapped onto the corresponding three-dimensional models respectively, the texture information resolutions of different parts of the finally generated model may be inconsistent, which affects the overall effect. Therefore, the two two-dimensional images may be pre-processed first to unify the resolutions of the two two-dimensional images, for example, the low-resolution image is upsampled to improve its resolution, and then the pre-processed two two-dimensional images are used to perform texture mapping processing on the three-dimensional model.

In some embodiments, considering that users often have different requirements when displaying the multiple streams of medical data, for example, at some times, a set of multiple streams of medical data collected by multiple device sources at a certain time point needs to be displayed, and at some times, only a single stream data collected by a certain device source at a certain time point needs to be displayed, and at some times, changes in multiple streams of data collected by a certain device source at multiple time points need to be displayed. In order to meet different requirements of the user and conveniently and quickly respond to the display instruction of the user. After the multiple streams of medical data collected by different device sources at the same time point are spatially aligned, and after the multiple streams of medical data collected by the same device source at different time points are spatially aligned, an expression model may be first generated based on the aligned data. The expression model may include multiple data layer levels, different data layer levels include medical data of different dimensions, and data of different data layer levels may be associated. After the expression model is generated, the aligned medical data may be displayed based on the expression model.

In some scenarios, after the multiple streams of medical data are aligned, other processing may be performed on these medical data based on actual needs. For example, in some embodiments, the multiple streams of medical data include three-dimensional data, semantic segmentation processing may be performed on the three-dimensional data to obtain a semantic segmentation result, and then the expression model is generated based on the semantic segmentation result and the data alignment result.

Taking an oral disease diagnosis and treatment scenario as an example, after the three-dimensional model of the oral cavity is obtained, semantic segmentation processing may be further performed on the three-dimensional model of the oral cavity to obtain a single tooth. Alternatively, semantic segmentation processing is further performed on the CBCT data of the oral cavity to obtain a maxilla or a mandible. Then, an expression model may be generated based on processing results of various processing, in other words, the expression model is a representation manner of processing results of various processing such as alignment, semantic segmentation, and texture mapping on the multiple streams of medical data, and after the expression model is used to represent these processing results, data of different dimensions may be displayed based on a display requirement of the user and the expression model.

In some embodiments, the multiple streams of medical data are medical data in an oral disease diagnosis and treatment scenario, and the expression model may be divided into the following data layer levels during data layer level division:
(1) Spatial 3D grid layer level, including grid data obtained by gridding three-dimensional data of the user's skull, that is, this layer level includes multiple 3D grids, and spatial coordinates corresponding to each 3D grid. When gridding the three-dimensional data of the skull, even division may be performed (sizes of minimum 3D grids obtained by division are consistent), or uneven division may be performed (sizes of minimum 3D grids obtained by division are inconsistent). For example, in some scenarios, a size of the minimum 3D grid may be adaptively adjusted, for example, for a region with dense data, the grid may be divided at a higher resolution, that is, the size of the minimum 3D grid obtained through division may be smaller. For a region with relatively sparse data, a grid may be divided at a lower resolution, that is, a size of the minimum 3D grid obtained through division may be larger. In this way, a position of each medical data in the data dense area can be accurately represented, and the calculation load can be reduced.
(2) Geometry layer level, including geometric morphology data of the user's oral cavity, such as morphology of teeth, morphology of skull, morphology of jaw bone, morphology of face, etc. The geometric morphology may include information such as a shape and a surface texture of each part in the oral cavity, and a spatial position of the geometric morphology data is represented by grid coordinates in the spatial 3D grid layer level. For example, some 3D grids in the spatial 3D grid layer level may be associated with the three-dimensional model of the teeth, and a position of the associated 3D grid is a position of the teeth in the entire skull region.
(3) Attribute layer level, including attribute data of the user's oral cavity, where a spatial position of the attribute data is represented by grid coordinates in the spatial 3D grid layer level; and for example, the attribute data (such as pathological data) may be associated with the grids in the 3D grid layer level, and a position of the associated 3D grid is a position of a part to which the attribute data belongs in the skull region.
(4) Semantic element layer level, including geometric morphology data and attribute data corresponding to each semantic entity in the user's oral cavity, and a spatial position of each semantic entity is represented by grid coordinates in the spatial 3D grid layer level. Each semantic entity may be obtained by performing semantic segmentation on collected three-dimensional data or two-dimensional data, for example, semantic segmentation processing may be performed on oral three-dimensional scanning data to obtain each tooth entity. Each tooth entity may be represented based on the oral geometric morphology data and the attribute data. Each semantic entity may be associated with a grid in the 3D grid layer level, and the position of the associated 3D grid is the position of the semantic entity in the skull region.

Certainly, in the embodiment of the present disclosure, the multiple streams of medical data are divided into the above four data layer levels based on the general requirements of the user when viewing the data, and in practical applications, the division may also be performed in other ways, and is not limited to the above listed four data layer levels.

In some embodiments, the geometric morphology data may include one or more of the following: three-dimensional scanning data of the user's oral cavity, three-dimensional scanning data of the user's face, CBCT data of the user's oral cavity, CBCT data of the user's skull, texture information of the user's oral cavity, and texture information of the user's face. The texture information of the user's oral cavity is aligned with the three-dimensional model corresponding to the three-dimensional scanning data of the user's oral cavity, and the texture information of the user's face is aligned with the three-dimensional model corresponding to the three-dimensional scanning data of the user's face. The three-dimensional scanning data and the texture information may be displayed through different layers, and when the geometric morphology data is displayed, a three-dimensional model without texture information or a three-dimensional model with texture information may be displayed based on user selection.

In some embodiments, the attribute data may include one or more of: two-dimensional image data, for example, an image of the user's oral cavity, an image of the user's face, cephalometric lateral films, etc., pathological data, and physiological slice data.

In some embodiments, the semantic entity may include one or more of: a single tooth, a maxilla, a mandible, etc.

In some embodiments, after the expression model is generated based on the processing results of various processing (alignment, semantic segmentation, etc.) on the multiple streams of medical data, some or all data layer levels in the expression model may be displayed based on the display instruction triggered by the user. For example, in some scenarios, the user only wants to view the geometric morphology data, that is, only the data of the geometry layer level may be displayed, and the user only wants to view the attribute data, that is, only the data of the attribute layer level may be displayed. In some embodiments, in order to more finely display the multiple streams of medical data, the medical data of each data layer level may be divided into multiple layers, and the user may select to display the medical data of one or more of the layers. For example, for the semantic element layer level, because the layer level includes geometric shape data and attribute data of multiple semantic entities, geometric shape data of each semantic entity may be divided into one layer, and attribute data of each semantic entity may also be divided into one layer. When the user selects only the layer corresponding to the geometric shape data of a certain semantic entity, only the geometric shape of the semantic entity is displayed. When the user selects a layer corresponding to geometric shape data and a layer corresponding to attribute data of a semantic entity, data of the two layers of the semantic entity may both be displayed. For example, a three-dimensional model of only a certain tooth or a three-dimensional model of only a certain tooth carrying texture information may be displayed.

Certainly, the layers may be specifically set based on an actual display requirement, and the layer(s) is displayed or hidden, so that the user can conveniently view medical data of different dimensions.

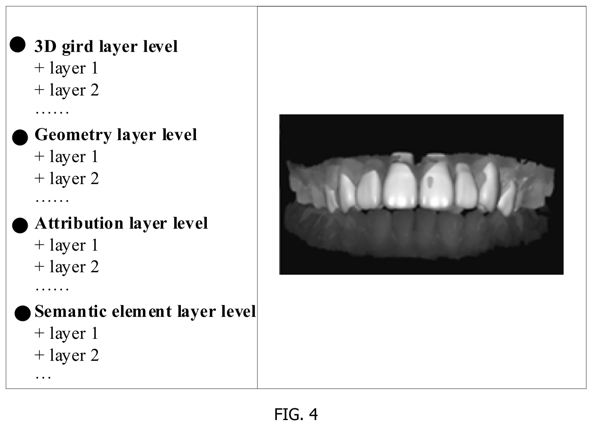

In some embodiments, as shown in FIG. 4, to facilitate user operation, multiple data layer level s of the expression model and layer identifiers of multiple layers at each data layer level may be displayed on the interaction interface. The user may select to display or hide these data layer levels or layers under each data layer level, and then display the medical data corresponding to the layer selected by the user on the interactive interface based on the user layer selection operation. In some embodiments, for a layer not selected by the user, the medical data corresponding to the layer may be transparently or semi-transparently displayed. For example, if the user wants to view the information of a certain tooth, that is, the user can select the corresponding layer of the tooth, at this time, if the layers corresponding to other teeth are set to a transparent state, the interactive interface will only display this single tooth. If the layers corresponding to other teeth are set to a semi-transparent state, the interactive interface will also display other teeth in a darker form, which is convenient for the user to understand the position of the current tooth in the entire oral cavity.

It is not difficult to understand that the solutions described in the above embodiments can be combined in a non-conflict basis, which are not illustrated in the embodiments of the present disclosure.

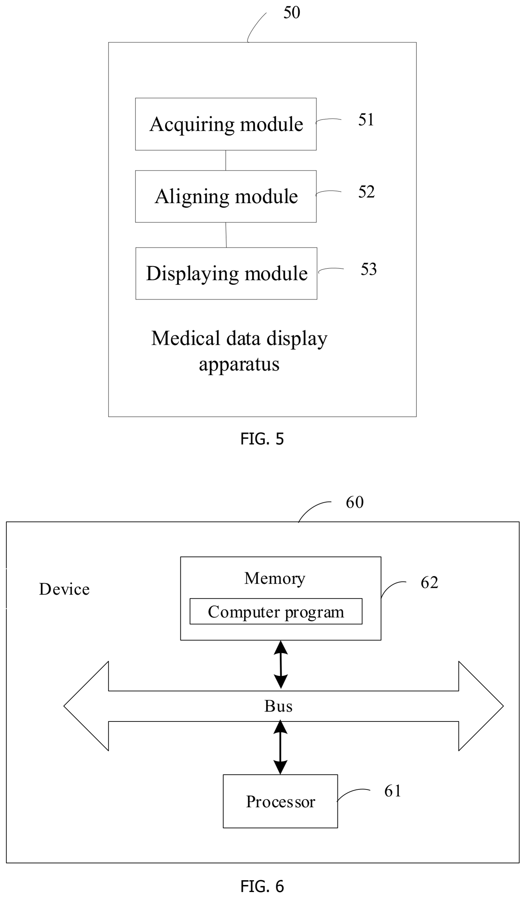

Correspondingly, an embodiment of the present disclosure further provides a medical data display apparatus 50, as shown in FIG. 5, including: an acquiring module 51, configured to acquire multiple streams of medical data collected by multiple device sources for a same target object at different time points respectively; an aligning module 52, configured to spatially align multiple streams of medical data collected by different device sources at a same time point, and spatially align multiple streams of medical data collected by a same device source at different time points; a display module 53, configured to, in response to acquiring an instruction triggered by a user for displaying medical data at a target time point, display, on an interactive interface, aligned multiple streams of medical data collected by different device sources at the target time point; and in response to acquiring an instruction triggered by the user for displaying medical data collected by a target device source, display, on the interactive interface, changes in medical data collected by the target device source at different time points.

For specific steps of performing the medical data display method by the apparatus, the descriptions of the foregoing method embodiments can be referred to, which will not be repeated herein.

Further, an embodiment of the present disclosure further provides a device, as shown in FIG. 6, the device includes a processor 61, a memory 62, and computer instructions stored in the memory 62 and executable by the processor 61, and the processor 61 implements the method according to any one of the above embodiments when executing the computer instructions.

An embodiment of the present disclosure further provides a computer-readable storage medium having a computer program stored thereon, where the program, when executed by a processor, implements the method according to any one of the foregoing embodiments.

The computer-readable medium includes permanent and non-permanent, and removable and non-removable media that can implement information storage by any manner or technology. The information may be a computer-readable instruction, a data structure, a program module, or other data. Examples of a computer storage medium include, but are not limited to, a phase change random access memory (PRAM), a static random access memory (SRAM), a dynamic random access memory (DRAM), another type of random access memory (RAM), a read-only memory (ROM), an electrically erasable programmable read-only memory (EEPROM), a flash memory or another memory technology, a compact disc read-only memory (CD-ROM), a digital versatile disc (DVD) or another optical storage, a magnetic cassette tape, a magnetic tape disk storage or another magnetic storage device, or any other non-transmission medium, and may be configured to store information that can be accessed by a computing device. As defined herein, the computer-readable medium does not include transitory computer-readable media, such as modulated data signals and carriers.

It can be seen from the description of the above embodiments that those skilled in the art can clearly understand that the embodiments of the present disclosure can be implemented by means of software plus a necessary general hardware platform. Based on such understanding, the technical solutions of the embodiments of the present disclosure in essence or the part contributing to the prior art may be embodied in the form of a software product, and the computer software product may be stored in a storage medium, such as a ROM/RAM, a magnetic disk, an optical disk, etc., including several instructions to cause a computer device (which may be a personal computer, a server, or a network device, etc.) to perform the methods described in various embodiments or some parts of the embodiments of the present disclosure.

The system, apparatus, module, or unit described in the foregoing embodiments may be implemented by a computer chip or an entity, or may be implemented by a product having a function. A typical implementation device is a computer, and a specific form of the computer may be a personal computer, a laptop computer, a cellular phone, a camera phone, a smartphone, a personal digital assistant, a media player, a navigation device, an email transceiver device, a game console, a tablet computer, a wearable device, or a combination of any several of these devices.

The various embodiments in this specification are described in a progressive manner, the same or similar parts between the various embodiments can be referred to each other, and each embodiment focuses on the differences from other embodiments. In particular, for the apparatus embodiment, since it is basically similar to the method embodiment, the description is relatively simple, and the relevant parts can be referred to the description of the method embodiment. The apparatus embodiments described above are merely illustrative, where the modules described as separate components may or may not be physically separated, and the functions of the modules may be implemented in the same or multiple software and/or hardware when implementing the solutions of the embodiments of the present disclosure. Some or all of the modules may also be selected according to actual needs to achieve the objectives of the solutions of the embodiments. Those skilled in the art can understand and implement without making creative efforts.

The above are only specific implementations of the embodiments of the present disclosure, and it should be noted that for those of ordinary skill in the art, several improvements and modifications can be made without departing from the principles of the embodiments of the present disclosure, and these improvements and modifications should also be considered as within the protection scope of the embodiments of the present disclosure.

### Industrial applicability

The medical data display method provided by the embodiments of the present disclosure can realize the display of multiple streams of medical data collected by different device sources at a certain time point and the display of changes in medical data collected by a certain device source at different time points on the interactive interface, which improves the data display efficiency, facilitating users to view data, and having strong industrial applicability.

## Claims

1. A medical data display method, comprising:
acquiring multiple streams of medical data collected by multiple device sources for a same target object at different time points respectively;
spatially aligning multiple streams of medical data collected by different device sources at a same time point, and spatially aligning multiple streams of medical data collected by a same device source at different time points;
in response to acquiring an instruction triggered by a user for displaying medical data at a target time point, displaying, on an interactive interface, aligned multiple streams of medical data collected by different device sources at the target time point; and
in response to acquiring an instruction triggered by the user for displaying medical data collected by a target device source, displaying, on the interactive interface, changes in medical data collected by the target device source at different time points.

2. The method according to claim 1, wherein displaying, on the interactive interface, the aligned multiple streams of medical data collected by the different device sources at the target time point comprises:
performing fusion processing on overlapping portions of the aligned multiple streams of medical data, and displaying fused multiple streams of medical data on the interactive interface.

3. The method according to claim 2, wherein displaying, on the interactive interface, the changes in the medical data collected by the target device source at different time points comprises:
displaying, by using animation, the changes in the medical data collected by the target device source at different time points, wherein an animation frame corresponding to a time point between two adjacent time points is generated by interpolation animation.

4. The method according to claim 1, wherein the multiple streams of medical data comprise three-dimensional data of the target object collected by a specified device source at different time points, and the method further comprising:
for three-dimensional data at each time point, performing semantic segmentation on the three-dimensional data to obtain multiple semantic entities;
spatially aligning the multiple semantic entities at different time points; and
in response to acquiring an instruction triggered by the user for displaying a target semantic entity, displaying, on the interactive interface, a change situation of the target semantic entity at different time points.

5. The method according to any one of claims 1-4, wherein spatially aligning the multiple streams of medical data collected by the different device sources at the same time point comprises at least one of:
spatially aligning at least two streams of three-dimensional data collected by different device sources at the same time point;
spatially aligning at least two two-dimensional images collected by different device sources at the same time point;
mapping texture of at least one two-dimensional image acquired by different device sources at the same time point to a three-dimensional model represented by at least one stream of three-dimensional data; or
aligning at least one stream of pathological data collected by different device sources at the same time point with at least one two-dimensional image or three-dimensional data.

6. The method according to claim 5, wherein the multiple streams of medical data are medical data in an oral diagnosis and treatment scenario, and spatially aligning the at least two streams of three-dimensional data comprises at least one of:
spatially aligning three-dimensional scanning data of a user's oral cavity and three-dimensional scanning data of a user's face;
spatially aligning three-dimensional scanning data of the user's oral cavity and cone-beam computed tomography (CBCT) data of the user's oral cavity; or
spatially aligning three-dimensional scanning data of the user's face and CBCT data of a user's skull.

7. The method according to claim 5 or 6, wherein the multiple streams of medical data are medical data in an oral diagnosis and treatment scenario, and mapping the texture of the at least one two-dimensional image to the three-dimensional model represented by the at least one stream of three-dimensional data comprises at least one of:
mapping texture of a two-dimensional image of a user's face to a three-dimensional model represented by three-dimensional scanning data of the user's face; or
mapping texture of a two-dimensional image of a user's oral cavity to a three-dimensional model represented by three-dimensional scanning data of the user's oral cavity.

8. The method according to any one of claims 5-7, wherein the multiple streams of medical data comprise at least two two-dimensional images collected in different illumination environments, and before mapping the texture of the at least two two-dimensional images to the three-dimensional model represented by the at least one stream of three-dimensional data, the method further comprising:
pre-processing the at least two two-dimensional images to unify brightness of the at least two two-dimensional images.

9. The method according to claim 5, wherein the at least two two-dimensional images comprise a two-dimensional image of a user's oral cavity and a two-dimensional image of a user's face.

10. The method according to claim 5, wherein the multiple streams of medical data comprise at least two two-dimensional images collected by photographing devices with different resolutions, and before mapping the texture of the at least two two-dimensional images to the three-dimensional model represented by the at least one stream of three-dimensional data, the method further comprising:
pre-processing the at least two two-dimensional images to unify resolutions of the at least two two-dimensional images.

11. The method according to claim 10, wherein the at least two two-dimensional images comprise a two-dimensional image of a specified part captured by a single-lens reflex camera and a two-dimensional image of the specified part collected by a three-dimensional scanning device, and the specified part comprises at least one of a user's face or a user's oral cavity.

12. The method according to claim 1, wherein after spatially aligning the multiple streams of medical data collected by the different device sources at the same time point, and/or spatially aligning the multiple streams of medical data collected by the same device source at different time points, the method further comprising:
generating an expression model based on the aligned data, wherein the expression model comprises multiple data layer levels, and different data layer levels comprise medical data of different dimensions; and
displaying the aligned medical data based on the expression model.

13. The method according to claim 12, wherein the multiple streams of medical data further comprise three-dimensional data of the target object, and the method further comprising:
performing semantic segmentation processing on the three-dimensional data to obtain a semantic segmentation result; and
wherein generating the expression model based on the aligned data comprises:
generating the expression model based on the aligned data and the semantic segmentation result.

14. The method according to claim 12, wherein the multiple streams of medical data are medical data in an oral diagnosis and treatment scenario, and the expression model comprises following data layer levels:
a spatial 3D grid layer level, comprising grid data obtained by gridding three-dimensional data of a user's skull;
a geometry layer level, comprising geometric morphology data of a user's oral cavity, wherein a spatial position of the geometric morphology data is represented by grid coordinates in the spatial 3D grid layer level;
an attribute layer level, comprising attribute data of the user's oral cavity, wherein a spatial position of the attribute data is represented by grid coordinates in the spatial 3D grid layer level; and
a semantic element layer level, comprising geometric morphology data and attribute data corresponding to each semantic entity in the user's oral cavity, and a spatial position of each semantic entity is represented by grid coordinates in the spatial 3D grid layer level.

15. The method according to claim 14, wherein the geometric morphology data comprises at least one of: three-dimensional scanning data of a user's oral cavity, three-dimensional scanning data of a user's face, CBCT data of the user's oral cavity, CBCT data of the user's skull, CBCT data of a user's jaw bone, texture information of the user's oral cavity, or texture information of the user's face.

16. The method according to claim 14 or 15, wherein the attribute data comprises at least one of: two-dimensional image data, pathological data, or physiological slice data.

17. The method according to any one of claims 14-16, wherein the semantic entity comprises at least one of: a single tooth, a maxilla, or a mandible.

18. The method according to claim 12, wherein displaying the aligned data based on the expression model comprises:
displaying medical data of some or all of the multiple data layer levels of the expression model based on a display instruction triggered by the user.

19. The method according to claim 12, wherein medical data of each of the data layer levels is able to be displayed through multiple layers, and the user is able to select to display the medical data of one or more of the layers.

20. The method according to claim 19, wherein displaying the aligned data based on the expression model comprises:
in response to a layer selection operation of the user on the interactive interface, displaying medical data corresponding to a layer selected by the user on the interactive interface, wherein the interactive interface displays multiple data layer levels of the expression model and layer identifiers of the multiple layers at each of the data layer levels.

21. The method according to claim 20, further comprising: for a layer not selected by the user, transparently or semi-transparently displaying medical data corresponding to the layer.

22. A medical data display apparatus, comprising:
an acquiring module, configured to acquire multiple streams of medical data collected by multiple device sources for a same target object at different time points respectively;
an aligning module, configured to spatially align multiple streams of medical data collected by different device sources at a same time point, and spatially align multiple streams of medical data collected by a same device source at different time points;
a display module, configured to, in response to acquiring an instruction triggered by a user for displaying medical data at a target time point, display, on an interactive interface, aligned multiple streams of medical data collected by different device sources at the target time point; and in response to acquiring an instruction triggered by the user for displaying medical data collected by a target device source, display, on the interactive interface, changes in medical data collected by the target device source at different time points.

23. An electronic device, comprising one or more processors, a memory, and a computer instruction stored in the memory and executable by the one or more processors, wherein when executing the computer instruction, the one or more processors are caused to perform the method according to any one of claims 1-21.

24. A computer-readable storage medium, storing a computer program, and when the computer program is executed by one or more processors, the one or more processors are caused to performed the method according to any one of claims 1-21.
